# EUROPÄISCHE PATENTSCHRIFT

(11) **EP 0 357 693 B1**
(45) Veröffentlichungstag und Bekanntmachung des Hinweises auf die Patenterteilung: **15.06.1994**
(21) Anmeldenummer: 89901048.2
(22) Anmeldetag: 06.01.1989
(51) Int. Cl.: B01D 39/00, B01J 20/28

(54) **MOLEKULARSIEBANORDNUNG**
MOLECULAR SIEVE ARRANGEMENT
AGENCEMENT DE TAMIS MOLECULAIRE

(30) Priorität: 07.01.1988 CH 26/88
(43) Veröffentlichungstag der Anmeldung: 14.03.1990
(73) Patentinhaber: H J L PROJECTS & DEVELOPMENTS LTD., 3186 Düdingen (CH)
(72) Erfinder: LAUBE, Hans-Jürgen, CH-8573 Siegershausen (CH)
(74) Vertreter: Gachnang, Hans Rudolf
(86) Internationale Anmeldenummer: CH8900002
(87) Internationale Veröffentlichungsnummer: WO8906156

(56) Entgegenhaltungen:
- GB-A- 2 005 019
- US-A- 4 081 402
- US-A- 4 309 247
- US-A- 4 606 824
- MEIER/OLSON "Atlas of Zeolite Structure Types", 2.Auflage, 1987, BUTTERWORTHS, London, Seite 24
- D.W.BRECK "Zeolite Molecular Zieves", 1974, JOHN WILEY&SONS, New York, Seiten 18,135,137,155 und 158

## Beschreibung

Gegenstand der vorliegenden Erfindung ist eine Molekularsiebanordnung gemäss Oberbegriff des Patentanspruches 1.
Gegenstand der Erfindung sind Verwendungen der Molekularsiebanordnungen gemäss den Ansprüchen 5 bis 11.

Natürliche und künstliche Zeolith-Molekularsiebe eignen sich zur Trennung von Mischungen anorganischer und organischer Stoffe, zur Entfernung unerwünschter Beimischungen aus Gasen und Flüssigkeiten. Eines der Verwendungsgebiete der Molekularsiebe ist heute die Trocknung von Gasen und Flüssigkeiten, beispielsweise Azeton, Butan, Toluol, etc.
Molekularsiebe werden auch verwendet zur Entfernung von Kohlenmonoxyd, Kohlenwasserstoff, Stickstoff, Methan, usw., aus Luft. Sie finden auch Verwendung in Ionenaustauschern.

Molekularsiebe im Sinne der Erfindung können aus natürlichen oder synthetischen Zeolithen bestehen, wobei synthetische Zeolithe häufig die gleiche Kristalstruktur wie natürliche Zeolithe aufweisen. Bei den natürlichen Zeolithen handelt es sich z.B. um Aluminosilicate, die die allgemeine Formel 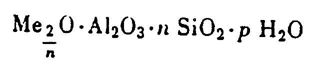
haben, wobei Me das Ion des Alkalimetalls (n = 1), meistens Na oder K, oder des Erdalkalimetalls (n = 2), gewöhnlich Ca, seltener Ba, Sr und Mg, bezeichnet. Weiter werden darunter die meisten Ad/Absorbensien verstanden.

Die Mechanik der Trennung Von Molekeln aus flüssigen oder gasförmigen Medien mittels Molekularsieben erfolgt durch Adsorption, wobei das Molekularsieb als Adsorbens wirkt und durch die Grösse und den energetischen Charakter seiner Oberfläche die Adsorption beeinflusst. Die Adsorption von Ionen, Molekülen oder Zusammenballungen von Molekülen und dergl. - den Adsorbaten - ist bedingt durch die energetischen Eigenschaften der Grenzschicht zwischen den beiden Phasen. Die adsorbierten Molekel belegen kontinuierlich die innere Oberfläche des Adsorbens und bedecken sie. Es tritt somit nach einer gewissen Zeit eine Sättigung ein, welche eine weitere Adsorption verhindert.

Für die bisher bekannten Trennprozesse wurden nur Molekularsiebe verwendet, deren Poren 3 Angström (30 nm) bzw. grösser als 3 Angström waren, um den Eintritt der aus Gasen oder Flüssigkeiten zu filtrierenden Molekel zu ermöglichen, bzw. um die riesige innere Oberfläche der Molekularsiebe nutzbar zu machen.

Die Zeolithe der natürlichen Analcit-Gruppe, deren Poren in der Grössenordung von 2,6 Angström sind, haben als Molekularsiebe keine Verwendung gefunden, weil die zu adsorbierenden, bzw. zu absorbierenden Moleküle wegen ihrer Grösse nicht in die Primärporen des Analcit-Zeolith eindringen können. Das kleinste Molekül, das Wassermolekül, misst nur ca. 2,9 Angström (vgl. MOLEKULARSIEBE von Otto Grubner, Pavel Jirü und Milos Ralek, VEB Deutscher Verlag der Wissenschaften, Berlin 1968, Seite 27).
Die aus dem Dokument "Zeolite Molekular Sieves" by Donald W. Beck bekannten Molekularsiebe Brewsterite und Hydroxy Sodalithe weisen Poren von 2,6 Angström (26 nm) Durchmesser auf und sind für die direkte Adsorption von NH₃ bzw. H₂O bestimmt.
Aus der US-A-4,606,824 ist eine Molekularsiebanordnung bekannt, bei der ein Molekularsiebmaterial in ein poröses Trägermaterial eingebettet ist. Gemäss der Beschreibung sollen durch den dort beschriebenen Filter Teilchen in der Grössenordnung von 1 bis 25 Millimikron ausgefiltert werden können. In einem weiteren Beispiel, Spalte 12, Zeilen 38 ff, liegt die Partikelgrösse bei 10 bis 100 Mikron. Es geht also hier um riesig grosse Teile im Vergleich zu denen, welche gemäss der vorliegenden Erfindung aus Gasen und Flüssigkeiten ausgeschieden werden sollen. Wie bekannt, ist ein Mikron zehntausendmal grösser als ein Angström. Es handelt sich also bei der US-Patentschrift um einen "Grobfilter", in dem die durchfliessende zu filtrierende Flüssigkeit von grossen Partikeln befreit und diese innerhalb der Poren des Molekularsiebmateriales zurückgehalten werden.
Die ad-/absorbierten Moleküle bleiben folglich ausserhalb der Poren, werden aber durch deren elektrostatischen Kräfte dort (am Eingang der Poren) festgehalten und zwar in den Poren des Trägermateriales.
Die weiter im Europäischen Recherchenbericht genannte US-A-4,081,402 betrifft ein Produkt, das aus einer Hydrogelmatrix besteht, in welcher vorzugsweise Aktivkohle feinst verteilt eingebaut ist. An Stelle von Aktivkohle können auch Molekularsiebe eingebaut sein, deren Porengrösse grösser ist als 3 Angström.

Die nachteiligen Eigenschaften der bekannten Molekularsiebe bestehen darin, dass deren innere Oberflächen grundsätzlich direkt beladen werden und mit einem Adsorptivanteil von ca. 25 %, je nach Molekularsiebtyp, dann keine weiteren Molekeln mehr aufgenommen werden können.

Die Beladung der inneren Oberflächen, der herkömmlichen Molekularsiebe erfolgt auch bereits beim Kontakt mit der Umgebungsluft bzw. durch die darin enthaltenen Wassermoleküle. Die Aufnahmekapazität ist folglich in doppelter Hinsicht beschränkt.

Hier schafft die Erfindung Abhilfe.
Die Erfindung, wie sie in den Ansprüchen gekennzeichnet ist, löst die Aufgabe, eine Molekularsiebanordnung zur Filtration von Molekeln oder mikroskopisch kleinen Partikeln aus Gasen oder Flüssigkeiten zu schaffen, bei der die inneren Oberflächen des Molekularsiebes weder durch Wassermoleküle noch durch die filtrierten Moleküle belegt werden können.

Es gelingt mit dem in einem Trägermaterial eingebauten Molekularsieb mit Porengrösse kleiner/gleich 2,6 Angström (26 nm) (Primärporen), die zu filtrierenden Molekel oder Partikel in die Poren- und Kanälestruktur des Trägermaterials hineinzubringen; die inneren Oberflächen des Molekularsiebes mit dessen Poren kleiner/gleich 2,6 Angström bleiben weitgehend frei und behalten die statische Ladung (Coulomb'sche Anziehung) bei.

Die Poren- und Kanäle-Struktur des Trägermaterials kann Querschnitte aufweisen, welche bis zu einem Faktor 1000 und mehr grösser sind als die Poren des Molekularsiebes. In diese verhältnismässig riesigen inneren Räume können entsprechend grössere Mengen von zu filtrierenden Teilchen eingelagert werden, bevor die statische Anziehung des Adsorbens nicht mehr genügt, weitere geladene Teilchen in die Kanälestruktur des Trägermaterials hineinzuziehen. Das im Trägermaterial vorhandene Wasser, das als Transportmedium dienen kann, wird durch einzulagernde geladenene Teilchen sukzessive aus den Kanälen verdrängt. Die Aufnahmefähigkeit hängt folglich zum einen von den Anziehungskräften des Zeolithmaterials ab; die Teilchengrösse der eingelagerten geladenen Teilchen hat keinen wesentlichen Einfluss auf die mengenmässige Kapazität.

Dieser Mechanismus bevorzugt somit polar hochwertige Teilchen und Partikel, z.B. Viren; niederwertigere Moleküle können dadurch aus der Kanälestruktur, bzw. über die Trägermaterial-Oberflächenporen wieder verdrängt werden. Es wird folglich auch eine Selektion möglich, die durch die geeignete Wahl der Kanälequerschnitte und Oberflächenporen des Trägermaterials, d.h. durch die Sekundärporen- und Kanälestruktur, gesteuert wird. Durch die Möglichkeit, Zeolithe mit Poren kleiner/gleich 2,6 Angström in der erfindungsgemässen Molekularsiebanordnung direkt in wässerigen Medien einzusetzen, können Viren oder allgemein Erreger, aus Flüssigkeiten, z.B. Blut oder Blutplasma, ausfiltriert werden.

Vorzugsweise wird beim Einsatz in Blut das Materialcompound aus Adsorbens- und porösem Trägermaterial mit Wasser vorbeladen, damit dem zu filtrierenden Medium kein Wasser, sondern einzig polar erfassbare Teilchen entzogen werden können. Bei einem Einsatz in gasförmigem Medium kann auch eine Vorbeladung der Trägermaterialhohlräume mittels eines inerten Gases erfolgen.

Ein weiterer Vorteil des erfindungsgemässen Materialcompounds ist die Einsparung an Energie bei der Desorption des Adsorptivs. Weil die eingelagerten Teilchen nur in der Kanälestruktur des Trägermaterials vorgelagert sind, nicht jedoch an den inneren Oberflächen des Zeolithmaterials adsorbiert werden, so kann mit wesentlich weniger Energie und folglich auch geringeren Temperaturen eine Regenerierung oder eine Ausspülung erfolgen. Die Wahl des Trägermaterials wird dadurch vereinfacht, d.h. es können auch Kunststoffe, z.B. in Form von Fasern als Trägermaterialien, mit riesiger Oberfläche und damit hoher Dynamik für wiederverwendbare Material-Compounds Verwendung finden.

In nicht brennbare, poröse Trägermaterialien, z.B. Stein, Bims, Tonerden, geschäumte Metalle, eincompoundierte Adsorbensien kleiner/gleich 2,6 Angström können zur Reinigung von Abgasen an Verbrennungsmaschinen eingesetzt werden, wobei eine Regenerierung durch Auswaschen möglich ist.

Im Vergleich zur Aktivkohle weist das erfindungsgemässe Materialcompound folglich eine wesentlich höhere Dynamik, Kapazität, einen geringeren Energieaufwand bei der Desorption (Regeneration) und zudem bessere Formstabilität auf. In Gestalt von Kapseln oder Tabletten kann das Materialcompound anstelle von Aktivkohle, z.B. zum Aufnehmen von im Magen-/Darmsaft vorhandenen Partikeln wie Viren, Bakterien und dgl. eingenommen werden.
Durch Beifügen von hartmagnetischen Zusätzen im Compound kann dieses nach Durchlaufen einer Transportstrecke im zu reinigenden Medium einfach zusammen mit den eingefangenen Partikeln wieder ausgeschieden werden.
Die grösste Dynamik der erfindungsgemässen Produktes wird in Form von Fasern erreicht, da diese eine pro Gewichtseinheit sehr grosse Oberfläche aufweisen und dennoch, z.B. in Gestalt eines Vlieses, einfach in flüssigen und gasförmigen Medien zu handhaben sind.
In der Gestalt von Faserschnitzeln, denen eine weitere Komponente beigegeben ist, kann das erfindungsgemässe Material in Zirkulation, z.B. in der Blutbahn eines Lebewesens, gebracht werden und anschliessend durch entsprechende Mittel, z.B. Magnetfelder, wenn die zusätzliche Komponente magnetisch erfassbar ist, wieder aus der Zirkulation ausgeschieden werden. Da die zusätzliche Komponente vorzugsweise selbst keine Anziehungskraft aufweist, können Zusammenballungen in der Zirkulationsbahn vermieden werden.
Durch das Einbinden des erfindungsgemässen Materialcompounds in vorzugsweise hygroskopische Flüssigkeiten, Salben oder Crèmes kann der Zutritt von Erregern zu Wundstellen verhindert und zugleich auf der Wundstelle befindliche Erreger dieser entzogen werden.

Die Erfindung wird anhand von Beispielen näher erläutert. Es zeigen:
- Figur 1: eine stark vergrösserte Faser mit darin eingebauten Zeolithen,
- Figur 2: eine synthetische Killerzelle (in Gestalt eines Faserschnitzels) in ca. zehntausendfacher Vergrösserung,
- Figur 3: einen Querschnitt durch eine Faser mit Zeolithteilchen mit Porengrösse grösser als 3 Angström (beladen),
- Figur 4: einen Querschnitt durch eine Faser mit Zeolithteilchen mit Porengrösse kleiner als 3 Angström (unbeladen) und
- Figur 5: eine vergrösserte Darstellung einer Pore bzw. eines Kanals in einer Faser gemäss Figur 4.

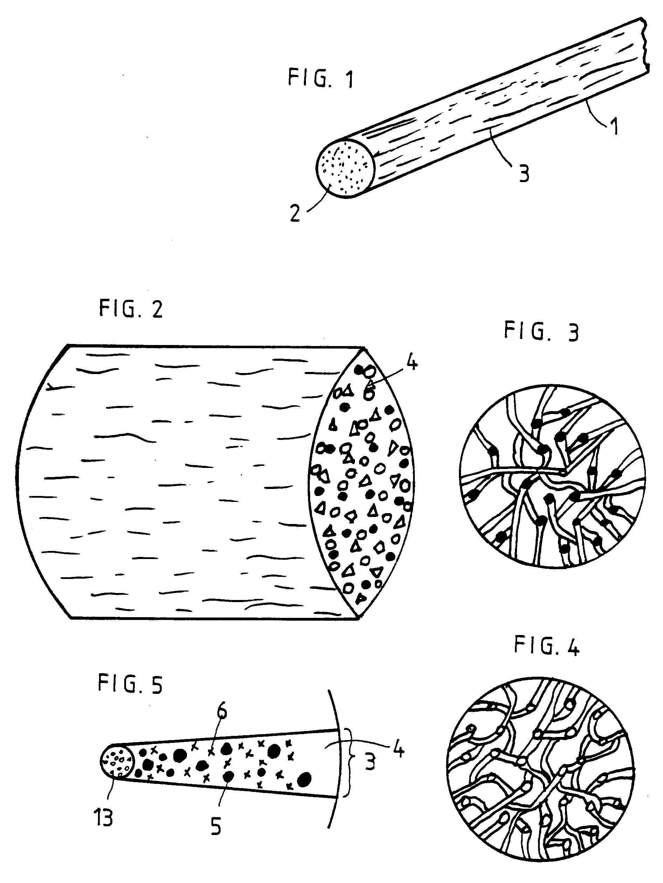

In Figur 1 ist ein Abschnitt einer Faser 1 dargestellt, in welcher Zeolithe 2 in Form von feinstem Pulver oder Granulat eingebaut sind. Der Einbau der Zeolithe 2 in die Trägerstruktur, d.h. in das Material, aus dem die Faser 1 hergestellt ist, ist nicht Gegenstand dieser Erfindung. Es bestehen verschiedene Möglichkeiten, das Zeolithteil 2 während des Spinnvorganges der Faser 1 beizufügen.
Die Faser 1 selbst besteht aus einem Material mit einer offenporigen Struktur. Diese ermöglicht die Aufnahme bzw. Abgabe von Wasser und Wasserdampf. Wasserdampf aufnehmende Fasern 1 ohne Zeolithe 2 sind bekannt; ein Verfahren zur Herstellung einer solchen Faser 1 wird z.B. in der EP-B1-0 029 949 und in der Zeitschrift Chemiefasern/Textilindustrie, 31/82 (1981, Seiten 112 - 116) erläutert.
Wenn in einer solchen Faser 1, deren Poren 3 und Kanäle 4 einen Querschnitt von ca. 1000 nm und Zeolithteile 2 mit Primärporengrösse grösser als 3 Angström eingebaut sind, so können damit Partikel 5 wie Viren, Bakterien und Moleküle 6 aus der Umgebung gefiltert werden, welche durch die Poren (Sekundärporen) 3 auf der Oberfläche des Trägermaterials in dieses eindringen können und von den Anziehungskräften der Zeolithe 2 angezogen werden. Da Wassermoleküle 6 mit ca. 2,9 Angström immer kleiner als die (Primär-) Poren 13 eines eingebauten herkömmlichen 3-Angström- oder grösser als 3-Angström-Zeolithtypen sind und durch die Poren 13 an die inneren Oberflächen der Zeolithe 2 gelangen, ist ein sinnvoller Einsatz in der freien flüssigen oder gasförmigen Phase in der freien Atmosphäre bzw. dem flüssigen Medium wegen kurzzeitiger Beladung mit Wassermolekülen nicht gegeben.

Die Anziehungskraft eines herkömmlichen Molekularsiebgranulatkorns nimmt durch die Einlagerung, d.h. Adsorption, von Molekülen über seine Primärporen 13 demzufolge bis zu einer maximalen Beladung, d.h. von bis zu ca. 25 Gewichtsteilen, in der freien Atmosphäre stetig und schnell ab.

Werden nun erfindungsgemäss, wie in Figur 4 dargestellt, Zeolithe 2 mit einer Porengrösse kleiner/gleich 2,6 Angström in die Faser 1 eingebaut, so können die durch die Kanäle 4 einer porösen Faser 1 eintretenden Molekel, Partikel, etc., nur bis zum Nahbereich der eingebauten Zeolithteilchen vordringen; ein Eintritt in die Primärporen 13 hingegen ist nicht möglich. Auch das Wassermolekül kann die Primärporendurchmesser (z.B. 2,6 Angström) des Zeolithes 2 der Analcytgruppe nicht beladen (vgl. Figur 5). Auf diese Weise bleibt die Anziehungskraft der Zeolithe 2 weitgehend aufrechterhalten, und die Sekundärporenstruktur des Trägermaterials, welche vorzugsweise im Nanometer/Mikronbereich gross gewählt wird, kann eine sehr grosse Menge von polar erfassbaren Teilchen, Molekeln, aber auch Bakterien, Viren, und dergl. aufnehmen und zurückhalten. Auch bei beladenen Trägermaterialkanälen 4 bleibt die Anziehungskraft der Zeolithkristalle erhalten, so dass an Polarität höherwertige Teilchen durch die z.B. mit Wasser beladenen Kanälestruktur diffundieren können. Der entsprechende Teil an Wasser wird nach aussen verdrängt. Die weitere Beladung hört erst auf, wenn ein Gleichgewicht zwischen Adsorbens und Adsorptiv eintritt.
Bindet man das Zeolithmaterial in Kunststoffe ein, so lässt sich das alkalische Zeolith-Material (PH-Wert ca. 11,5) auch in alkaliempfindlichen Substanzen verwenden, da ein direkter Kontakt mit dem zu reinigenden Medium, z.B. im Nahrungsmittelbereich und Hygienebereich, nun verhindert wird. Als Trägerstoff eignet sich dazu beispielsweise Polyamid, Polyacryl, aber auch Aktivkohle; für hitzbeständige Verwendungen auch Erden oder Metalle.

Wie bereits oben angedeutet, wird es nun möglich, z.B. Bakterien von einer Grösse von z.B. 2 Mikron oder Viren mit einer Grösse von z.B. zwischen ca. 8 und 400 nm, aus Flüssigkeiten oder aus der Gasphase zu trennen. Insbesondere die Viren weisen eine stark geladene Oberfläche auf und werden dadurch von der Ladung der im Trägermaterial eingebundenen Adsorbensien in die (sekundäre) Kanälestruktur des Trägermaterials hineingezogen. Da die Wassermoleküle nicht in die Primärporen des Zeoliths eintreten können, verzögern sich die Viren in der Kanälestruktur des Trägermaterials. Die Wassermoleküle werden nachfolgend sukzessive wieder durch die vom Zeolith angezogenen Teilchen aus der Kanälestruktur des Trägermaterials über deren Oberflächenporen verdrängt. Das Wasser dient demzufolge als Transportmedium. Es wird möglich, durch ein mit Wasser vorbeladenes Trägermaterial verschiedenste Partikel, Bakterien und Viren, auch Pestizide aus der Umgebung anzuziehen und einzulagern.
Es ist beispielsweise möglich, einen Mund- und Nasenschutz mit aus Fasern und der erfindungsgemässen Molekularsiebanordnung hergestellten Vlies als Filter gegen das Eindringen von Viren und Bakterien usw. herzustellen und damit die Infektionen durch die Atemwege (Influenza, Schnupfen, Adeno- und Corona- wie Ruthinfektionen) und weitere Infektionen (Pocken, Röteln, Wildpocken, Mumps, Masern, etc.) zu verhindern. Durch die genannten Filter werden die an Tröpfchen, die beim Niessen entstehen, gebundenen Viren aufgefangen und zurückgehalten.
Die erfindungsgemässe Textilfaser, Watte oder das Vlies kann als Wattebausch, als Atemschutzmaske, aber auch als Filter in Belüftungs- und Klimaanlagen eingesetzt werden.

Da die alkalischen Eigenschaften des Zeolithmaterials durch den Einbau in ein poröses Trägermaterial keinen Einfluss mehr auf die zu reinigenden Stoffe haben, ist es möglich, Blut und Plasma von darin enthaltenen Partikeln, wie Bakterien, Viren, etc., zu reinigen, indem letzteres durch einen mit entsprechend präpariertem Fasermaterial gefüllten Schlauch geleitet und dem menschlichen oder tierischen Körper nach dem Durchleiten wieder zugeführt wird.

Wird dem Compound zusätzlich noch hartmagnetisches Metallmaterial beigemengt, z.B. (MeCO3 + FeO3), so können die in der zu reinigenden Flüssigkeit zirkulierenden Compoundteilchen, z.B. in Gestalt von Faserschnitzeln lokalisiert und auch wieder ausgeschieden werden. Diese Faserschnitzel haben folglich die Wirkung einer Killerzelle, die mit ihr in der Flüssigkeit mitschwimmende Partikel (Viren, Bakterien etc.) einfangen. Eine solche Killerzelle kann beispielsweise aus porösen Material hergestellten Faserschnitzeln nach der erfindungsgemässen Molekularsiebanordnung bestehen. Die Poren (Primärporen) des synthetischen Zeolithkristalls weisen einen Durchmesser von z.B. 2,6 Angström auf. Die synthetische Faser besitzt eine Porenstruktur mit einem Durchmesser von z.B. 1000 nm. Das Faserschnitzel selbst weist einen Durchmesser von etwa 8 Mikron und eine Länge von 8 bis 10 Mikron auf, eine Grösse, die etwa einem T4-Helferlymphozyten entspricht. Als hartmagnetisches Material kann beispielsweise, wie oben bereits angeführt, MeCO3 und FeO3, zugesetzt werden, und zwar in Form von vorgebranntem oder vorgesintertem und zu einer Teilchengrösse von etwa 0,2 Mikron gemahlenem Werkstoff.
Diese Faserschnitzel können einer Flüssigkeit, z.B. in die Blutbahn oder Blutplasmakonserven, eingeschleust und nach einer gewissen Zeit mittels magnetischer Felder wieder ausgeschieden werden.

Zum Ausscheiden kann ein Faservliesmaterial gleicher Materialzusammensetzung wie die Faserschnitzel mit hartmagnetischen Teilchen (und somit erfassbar) verwendet werden, wobei das Faservliesmaterial jedoch zusätzlich magnetisiert wurde und beim Durchfluss die magnetisch erfassbaren Faserschnitzel zurückhält.

Der Einsatz ist nicht nur in organischen Flüssigkeiten, sondern auch zur Filtrierung von flüssigen Nahrungsmitteln möglich, wobei die Fasern/Faserschnitzel, wenn nötig, vor deren Einsatz mit Wasser steril vorgeladen werden.

Die eben beschriebenen synthetischen Killerzellen können zur Isolierung von bekannten und unbekannten Erreger/Virustypen benutzt werden, wenn diese nach Durchlaufen der Blutbahn, beispielsweise durch Vakuumtechnik aus dem Materialcompound extrahiert werden. Selbstverständlich können synthetische Killerzellen auch lokal in das lymphatische System eingebracht werden. Zum besseren Verständnis der Grössenverhältnisse zeigt Figur 2 in ca. zehntausendfacher Vergrösserung eine synthetische Killerzelle.

Mit den beschriebenen Verwendungen zum Einfangen von Viren, Bakterien und dergl. kann der Materialcompound in entsprechender Darreichungsform, z.B. mit einem hitzefesten Trägermaterial wie Tonerde, etc., zur Reinigung von Abgasen von Verbrennungsmotoren eingesetzt werden, z.B. am Ausgang des Schalldämpfers oder als separates Austauschteil.
Filter aus dem erfindungsgemässen Materialcompound können auch in Klima- und Lüftungsanlagen von Gebäuden und Fahrzeugen, auch Staubsaugern im Haushalt, eingesetzt werden und ermöglichen das Ausfiltrieren nicht nur von Staubpartikeln, sondern auch von gesundheitsschädlichen Erregern (Viren, Bakterien etc.), da deren Zeolithmaterialanteil mit seinen Primärporen von ca. 2,6 Angström nicht durch die allgegenwärtigen Wassermolekülen direkt beladen und deren Wirkung aufgehoben wird.
Ein Einsatz ist auch möglich als Einlage in Schuhen zur Verhinderung der Einlagerung von Sporen, die zu Fusspilz führen können.
Weiter besteht die Möglichkeit, den Materialcompound zur Lufttrocknung in Fahrzeugluftdruckbremsen einzusetzen, wodurch gegenüber dem herkömmlichen Zeolithgranulat mit einer Primärporengrösse ab 3 Angström, eine etwa vierfache Aufnahmekapazität an Feuchtigkeit möglich ist, bzw. mit einem wesentlich geringeren Volumen gearbeitet werden kann.

Ein weiterer Verwendungsbereich ist z.B. in Gestalt eines Faserzopfes, dessen Fasern aus dem Materialcompound hergestellt sind, in Isolierglasabstandhaltern.
Mit dem Materialcompound hergestellte Einsätze in Verschlüssen von Behältnissen oder an deren inneren Oberflächen dienen dazu, den Inhalt vor Feuchtigkeit zu schützen, bzw. den Inhalt zu entfeuchten.

Eine weitere Verwendung des erfindungsgemässen Materialcompounds in Gestalt von Fasern ist die Herstellung von Filtermatten, Operations- und Taschentüchern, Windeln und anderen Hygieneartikeln. Die im Taschentuchmaterial (Zellulose) eingearbeiteten Fasern entziehen der auf dieses aufgebrachten Feuchtigkeit die ansteckenden Partikel, wie Viren, etc. Die herkömmliche infektiöse Verbreitung durch das Tuch beim Wiedergebrauch wird dadurch sehr stark verringert. Analog können auch andere Tücher und Tampons im Hygienebereich mit der erfindungsgemässen Faser angereichert werden.

Bei grosstechnischen Anlagen können durch den Einsatz des Materialcompounds aus Wasser darin enthaltene Pestizide und dgl. gefiltert werden, wobei eine Regeneration des Filtermaterialcompounds, wie bereits oben erwähnt, mit geringem Energieaufwand möglich ist.

Eine weitere Verwendungsmöglichkeit des Materialcompounds ist das Einbinden von Viren auf Pflanzen. Wird das Materialcompound als Faserschnitzel z.B. mit Früchtewachs vermischt und durch Sprühen auf Pflanzen aufgebracht, so werden die Pflanzen vor Erregern, z.B. Tabakmosaik-Stäbchen, Viren von Tabakpflanzen, geschützt.

## Patentansprüche

1. Molekularsiebanordnung zur Filtration von Molekeln oder mikroskopisch kleinen Partikeln und Teilchen aus Gasen und/oder Flüssigkeiten, bei der das Molekularsiebmaterial in eine poröse Trägermaterialstruktur eingebunden ist, dadurch gekennzeichnet, dass die Primärporen des Molekularsiebmaterials eine Grösse aufweisen, die kleiner/gleich 2,6 Angström (26 nm) ist, so dass eine Ad/Absorption von Wassermolekülen unterbleibt und keine direkte Beladung an den inneren Oberflächen des Molekularsiebmaterials durch die zu filtrierenden Molekel oder mikroskopisch kleinen Partikel und Teilchen stattfindet, und daß das Trägermaterial eine aus Sekundärporen und Kanälen gebildete Struktur aufweist, in der die vom Molekularsiebmaterial angezogenen Molekel oder mikroskopisch kleinen Partikel und Teilchen aufgenommen und von der Anziehungskraft des unbelegten Molekularsiebmaterials zurückgehalten werden.

2. Molekularsiebanordnung nach Anspruch 1, dadurch gekennTrägermaterial mit einer Kanäle- und Porengrösse grösser als 3 Angström eingebaut ist.

3. Molekularsiebanordnung nach einem der Ansprüche 1 oder 2, dadurch gekennzeichnet, dass das Trägermaterial faserförmig ausgebildet ist.

4. Molekularsiebmaterial nach einem der Ansprüche 1 bis 3, dadurch gekennzeichnet, dass das Trägermaterial aus einem hitzebeständigen Stoff besteht.

5. Verwendung einer Molekularsiebanordnung nach einem der Ansprüche 1 bis 4 in einem flüssigen oder gasförmigen Medium zum Filtrieren und/oder Selektionieren von darin enthaltenen Molekülen, Partikeln und andern Teilchen.

6. Verwendung einer Molekularsiebanordnung nach einem der Ansprüche 1 bis 5, bei der die Poren im Trägermaterial eine kanalartige Struktur aufweisen.

7. Verwendung einer Molekularsiebanordnung nach einem der Ansprüche 1 bis 4 als Filter für flüssige oder gasförmige Medien in Flüssigkeits- oder Gasströmen zum Ausscheiden von darin enthaltenen Partikeln.

8. Verwendung einer Molekularsiebanordnung nach einem der Ansprüche 6 oder 7 zum Ausscheiden von Viren, Bakterien und dgl. aus Blut oder Blutplasma.

9. Verwendung einer Molekularsiebanordnung nach einem der Ansprüche 1 bis 4 in textilen Gebilden wie Matten, Operations- und Taschentüchern, Verbandstoffen, Windeln und Hygieneartikeln.

10. Verwendung einer Molekularsiebanordnung nach einem der Ansprüche 1 bis 4 in hygroskopischen Flüssigkeiten, Pasten oder Crèmes zum Reinigen von Wunden oder als Pflanzenschutz zum Aufbringen auf der Oberflache der Blätter zum Fernhalten von Erregern aus der Umgebungsluft.

11. Verwendung einer Molekularsiebanordnung nach einem der Ansprüche 1 bis 4 als Tabletten oder Kapseln zum Einnehmen zur Aufnahme von Partikeln, Viren, Bakterien und dgl. aus dem Magensaft

## Claims

1. Molecular sieve arrangement for filtration of molecules or microscopically small particles and particles comprising gases and/or liquids, in which the molecular sieve material is integrated into a porous carrier material structure, characterised in that the primary pores of the molecular sieve material are of a size smaller than or equal to 2.6 Angstroms (26nm), so that adsorption/absorption of water molecules is prevented, and no direct charging takes place on the inner surfaces of the molecular sieve material by the molecules or microscopically small particles to be filtered, and in that the carrier material has a structure formed from secondary pores and ducts, in which the molecules or microscopically small particles attracted by the molecular sieve material are taken in and held by the attractive power of the uncoated molecular sieve material.

2. Molecular sieve arrangement according to claim 1, characterised in that the molecular sieve arrangement is built into a porous carrier material with a duct and pore size of more than 3 Angstroms.

3. Molecular sieve arrangement according to one of claims 1 or 2, characterised in that the carrier material is fibrous in form.

4. Molecular sieve arrangement according to one of claims 1 to 3, characterised in that the carrier material comprises a heat-resistant material.

5. Utilisation of a molecular sieve arrangement according to one of claims 1 to 4 in a liquid or gaseous medium for filtering and/or selection of molecules and other particles contained therein.

6. Utilisation of a molecular sieve arrangement according to one of claims 1 to 5, in which the pores in the carrier material have a duct-like structure.

7. Utilisation of a molecular sieve arrangement according to one of claims 1 to 4 as a filter for liquid or gaseous media in liquid or gas streams, in order to separate particles contained therein.

8. Utilisation of a molecular sieve arrangement according to on of claims 6 or 7, for separating viruses, bacteria and the like from blood or plasma.

9. Utilisation of a molecular sieve arrangement according to one of claims 1 to 4 in textile formations such as matting, operating and hand cloths, dressing materials, nappies and articles of hygiene.

10. Utilisation of a molecular sieve arrangement according to one of claims 1 to 4 in hygroscopic liquids, pastes or creams for cleaning wounds or as plant protection for application to surface of the leaves in order to prevent their contact with exciters from the environmental air.

11. Utilisation of a molecular sieve arrangement according to one of claims 1 to 4 as tablets or capsules, to be taken internally, for receiving particles, viruses, bacteria and the like from the digestive juices.

## Revendications

1. Agencement de tamis moléculaire, pour la filtration de molécules ou petites particules et fragments de matière microscopiques faisant partie de gaz et/ou de liquides, dans lequel la matière de tamis moléculaire est intégrée dans une structure de matière de support poreuse, caractérisé en ce que les pores principaux de la matière de tamis moléculaire ont une taille qui est inférieure ou égale à 2,6 angströms (26 nm), de sorte qu'il ne se produit pas d'adsorption/absorption de molécules d'eau et qu'aucune fixation directe des molécules ou petites particules et fragments de matière microscopiques à filtrer n'a lieu sur les surfaces intérieures de la matière de tamis moléculaire, et en ce que la matière de support présente une structure, formée de pores secondaires et de canaux, dans laquelle les molécules ou petites particules et fragments de matière microscopiques ayant subi l'attraction de la matière de tamis moléculaire sont logés et sont retenus par la force d'attraction de la matière de tamis moléculaire non occupée.

2. Agencement de tamis moléculaire suivant la revendication 1, caractérisé en ce que la matière de tamis moléculaire est intégrée dans une matière de support poreuse ayant une taille de canaux et de pores supérieure à 3 angströms.

3. Agencement de tamis moléculaire suivant l'une des revendications 1 et 2, caractérisé en ce que la matière de support se présente sous forme de fibres.

4. Agencement de tamis moléculaire suivant l'une des revendications 1 à 3, caractérisé en ce que la matière de support est en une substance résistant à la chaleur.

5. Application d'un agencement de tamis moléculaire suivant l'une des revendications 1 à 4, dans un milieu liquide ou gazeux en vue de filtrer et/ou de sélectionner des molécules, particules et autres fragments de matière qui y sont contenus.

6. Application d'un agencement de tamis moléculaire suivant l'une des revendications 1 à 4, dans laquelle les pores situés dans la matière de support ont une structure en forme de canaux.

7. Application d'un agencement de tamis moléculaire suivant l'une des revendications 1 à 4 en tant que filtre pour des milieux liquides ou gazeux se trouvant dans des écoulements liquides ou gazeux, en vue de la séparation de particules qui y sont contenues.

8. Application d'un agencement de tamis moléculaire suivant l'une des revendications 6 et 7 à la séparation de virus, bactéries et analogues à partir du sang ou du plasma sanguin.

9. Application d'un agencement de tamis moléculaire suivant l'une des revendications 1 à 4 dans des produits textiles tels que des non-tissés, des champs opératoires et des mouchoirs, des matières pour pansement, des couches et des articles d'hygiène.

10. Application d'un agencement de tamis moléculaire suivant l'une des revendications 1 à 4 dans des liquides, pâtes ou crèmes hygroscopiques, en vue de nettoyer des blessures ou en tant que produit phytosanitaire à déposer sur la surface des feuilles en vue d'éloigner les agents pathogènes provenant de l'air environnant.

11. Application d'un agencement de tamis moléculaire suivant l'une des revendications 1 à 4 en tant que comprimés ou capsules en vue d'une administration permettant d'extraire du suc gastrique les particules, virus, bactéries ou analogues.
